**Europäisches Patentamt**

**European Patent Office**

**Office européen des brevets**

(19)

(11) Publication number : **0 479 454 A1**

# (12) EUROPEAN PATENT APPLICATION

(21) Application number : **91308585.8**

(51) Int. Cl.⁵ : **C07K 15/00, C12P 21/08**

(22) Date of filing : **20.09.91**

The applicant has filed a statement in accordance with Rule 28 (4) EPC (issue of a sample only to an expert). Accession number(s) of the deposit(s): HB-10541, HB-10542, HB-10543 and HB-10540.

(30) Priority : **24.09.90 US 587188**

(43) Date of publication of application :
**08.04.92 Bulletin 92/15**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Emini, Emilio A.**
**6 Faggs Manor Lane**
**Paoli, PA 19301 (US)**
Inventor : **Schleif, William A.**
**RD No. 1, Box 154**
**Green Lane, PA 18054 (US)**
Inventor : **Long, William J.**
**929 Tricorn Drive**
**Lansdale, PA 19446 (US)**

(74) Representative : **Barrett-Major, Julie Diane et al**
**Merck & Co., Inc. European Patent Department Terlings Park Eastwick Road Harlow Essex CM20 2QR (GB)**

(54) **HIV-neutralizing rat monoclonal antibodies against PB-1.**

(57) The preparation and characterization of rat monoclonal antibodies against a major type-specific neutralizing determinant of HIV are disclosed. The antibodies are useful in the diagnosis of AIDS and ARC, in passive immunization against exposure to HIV and in related immunotherapies against AIDS or ARC. The rat hybridomas of this invention are useful for humanization of the rat monoclonal antibodies.

**EP 0 479 454 A1**

Jouve, 18, rue Saint-Denis, 75001 PARIS

## BACKGROUND OF THE INVENTION

Acquired Immune Deficiency Syndrome (AIDS) is the clinical manifestation of the apparent infection of CD4 helper T-cells and other cell targets by human immunodeficiency virus (HIV), also previously referred to as human T-lymphotropic virus type III (HTLV-III), Lymphoadenopathy-associated virus (LAV), or AIDS-related virus (ARV) (hereinafter collectively "HIV"). AIDS is a transmissible deficiency of cellular immunity characterized by opportunistic infections and certain malignancies. A similar disease, AIDS-related complex (ARC), shares many of the epidemiological features and immune abnormalities with AIDS, and often precedes the clinical manifestations of AIDS.

Many of the details of the genetic function and virion structure of HIV have not yet been elucidated. However, certain general features have emerged. An RNA virus with a genome totaling about 9 kilobases (kb), its nucleotide sequence contains seven major open reading frames (ORFs) corresponding to the gag, pol and env, vif, tat, rev, and nef genes. The genes gag, pol and env code respectively for core subunits, viral enzymes such as reverse transcriptase or protease, and outer surface subunits. The gene vif codes for a viral infectivity factor, which is a protein involved with enhancement of cell-to-cell transmission of virions without affecting the budding process. The gene tat codes for a small protein required for viral infectivity, but its mechanism is not clear. The gene rev regulates expression of the viral proteins of gag, pol and env genes, possibly by facilitating transport of incompletely spliced RNA. The nef gene codes for a viral protein found in the cell cytoplasm, and it may modulate the host cellular signaling system and serve as a transcriptional silencer. Terminal repeats in the nucleotide sequence are common to many retroviruses such as HIV and are required for viral replication and integration into the host chromosome.

AIDS is a disease of a virus with a unique collection of attributes. HIV itself targets the immune system; it possesses a reverse transcriptase capable of turning out highly mutated progeny; it is sequestered from the immune system and it has a hypervariable surface in the (env) region. See, e.g Hilleman, M.R., Vaccine 6, 175 (1988); Barnes, D.M., Science 240, 719 (1988).

A monoclonal antibody specific for a major neutralizing determinant (MNtD) of HIV is an ideal prophylactic treatment for preventing the debilitating effects of infection by HIV. For example, virus-neutralizing monoclonal antibodies of this invention are delivered intravenously immediately following exposure to HIV, thereby rapidly achieving a virus-neutralizing immune state, as well as preventing the initial progression of HIV infection and the establishment of a persistent/latent infection.

Preliminary attempts in applicants' laboratory to produce mouse monoclonals with these characteristics failed. Instead, applicants have discovered, isolated and characterized a variety of rat monoclonal antibodies with these characteristics including HIV neutralization. These rat monoclonal antibodies are useful for immunotherapy or immunoprophylaxis of AIDS or ARC, including passive immunization and related immunological treatments.

Rat monoclonal antibodies are known to be difficult and impractical to prepare, particularly in view of the low efficiency of fusion. One advantage of the present invention is to show how such rat monoclonals are made. Another advantage provides a larger animal than the typical mouse, as more cells can be obtained for the purposes of cell fusion. Further, it was found that mice do not recognize the antigen used in the present application. Hence, an additional advantage is to provide monoclonal antibodies specific for the antigens not recognized by the mouse immunological system.

Therapeutic administration of monoclonal antibodies having neutralizing activity can be precisely adjusted for maximum benefit, unlike polyclonal antisera. Also, monoclonal antibodies can effectively neutralize HIV at lower concentrations than polyclonal preparations of similar binding specificities.

Because of their origin from a single cell, monoclonal antibodies and their hybridoma cells provide highly specific, uniform antibody preparations of any desired quantity.

## BRIEF DESCRIPTION OF THE INVENTION

The rat hybridoma cell lines of the present invention synthesize monoclonal antibody that binds PB1 protein. These monoclonal antibodies are useful in the passive immunization against exposure to HIV and in related immunotherapies for AIDS or ARC.

## ABBREVIATIONS AND DEFINITIONS

| | |
|---|---|
| AIDS | Acquired immune deficiency syndrome |
| ARC | AIDS-related complex |
| HIV | Generic term for the presumed etiological agent of AIDS and/or ARC, also referred to as strains HTLV-III, LAV, and ARV. |
| MNtD | Major neutralization determinant of HIV |
| Recombinant protein | A polypeptide or oligopeptide expressed by foreign DNA in a recombinant eukaryotic or procaryotic expression system. |
| Recombinant expression system | A cell containing a foreign DNA expressing a foreign protein or a foreign oligopeptide. |

### Amino Acids

| Full Name | Three-letter symbol | One-letter symbol |
|---|---|---|
| Alanine | Ala | A |
| Arginine | Arg | R |
| Asparagine | Asn | N |
| Aspartic acid | Asp | D |
| Asn and/or Asp | Asx | B |
| Cysteine | Cys | C |
| Glutamine | Gln | Q |
| Glutamic acid | Glu | E |
| Gln and/or Glu | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The terms "protein," "peptide," "oligopeptide," and "polypeptide" and their plurals have been used interchangeably to refer to chemical compounds having amino acid sequences of five or more amino acids. "Amino acid" refers to any of the 20 common amino acids for which codons are naturally available, and are listed in the table of amino acids given above. Combinations of substituents and/or variables are permissible only if such combinations result in stable compounds.

The present invention encompasses rat monoclonal antibodies capable of neutralizing HIV of the IIIB serotype. The antibodies of the present invention have a specificity for PB1 protein, of the amino acid sequence:

```
LeuAsnGlnSerValGluIleAsnCysThrArgProAsnAsnAsnThrArgLys
SerIleArgIleGlnArgGlyProGlyArgAlaPheValThrIleGlyLysIle
GlyAsnMetArgGlnAlaHisCysAsnIleSerArgAlaLysTrpAsnAsnThr
LeuLysGlnIleAspSerLysLeuArgGluGlnPheGlyAsnAsnLysThrIle
IlePheLysGlnSerSerGlyGlyAspProGluIleValThrHisSerPheAsn
CysGlyGlyGluPhePheTyrCysAsnSerThrGlnLeuPheAsnSerThrTrp
PheAsnSerThrTrpSerThrLysGlySerAsnAsnThrGluGlySerAspThr
IleThrLeuProCysArgIleLysGlnIleIleAsnMetTrpGlnGluValGly
LysAlaMetTyrAlaProProIleSerGlyGlnIleArgCysSerSerAsnIle
ThrGlyLeuLeuLeuThrArgAspGlyGlyAsnSerAsnAsnGluSer .
```

Rat hybridoma cell lines that produce these antibodies are also disclosed. The monoclonal antibodies of the present invention are useful in immunoprophylaxis and immunotherapy of AIDS or ARC. Further, the monoclonals of the present invention are useful in treating infection by HIV after suspected past exposure to HIV, e.g., by blood transfusion, accidental needle stick, or exposure to patient blood during surgery. Treatment of AIDS babies or infants infected with HIV by their mothers is another use of the monoclonal antibodies of the present invention.

Applicants have attempted without success to obtain mouse monoclonal antibodies to PB1. They now have succeeded with the rat system, which is generally known to be difficult and hard to work with. Applicants disclose a method for making rat monoclonal antibodies.

The particular antigen used, PB1, contains the "RP loop" sub sequence RGPGRAF specific for the IIIb serotype of the HIV envelope protein (residues 24-30). Since the specificity is for IIIB HIV infections, the monoclonal antibodies of the present invention are useful for patients infected with IIIB HIV. Preferably, these antibodies would be part of a cocktail of monoclonal antibodies each specific for a different subset of HIV envelope protein serotypes.

## Preparation of PB1

### A. Organic Synthesis of PB1:

Standard and conventional methods exist for rapid and accurate synthesis of PB1 on solid-phase supports. Solution-phase synthesis is also feasible.

Synthesis on solid-phase supports, or solid-phase synthesis, is most conveniently performed on an automated peptide synthesizer according to e.g., Kent, S. et al., "Modern Methods for the Chemical Synthesis of Biologically Active Peptides," in Alitalo, K. et al., (eds.). Synthetic Peptides in Biology and Medicine, Elsevier 1985, pp. 29-57. Manual solid-phase synthesis may be employed instead, by following the classical Merrifield techniques, as described, for example, in Merrifield, R.B. J. Am. Chem. Soc. 85, 2149 (1963), or known improvements thereof. Solid-phase peptide synthesis may also be performed by the Fmoc method, which employs very dilute base to remove the Fmoc protecting group. Segment synthesis-condensation is a further variant of organic synthesis of peptides as within the scope of the techniques of the present invention.

In organic synthesis of peptides, protected amino acids are condensed to form amide or peptide bonds with the N-terminus of a growing peptide. Condensation is usually performed with the carbodiimide method by reagents such as dicyclohexyl-carbodiimide, or N-ethyl, $N_1$-($\gamma$-dimethylaminopropyl) carbodiimide. Other methods of forming the amide or peptide bond include, but are not limited to, synthetic routes via an acid chloride, azide, mixed anhydride or activated ester. Common solid-phase supports include polystyrene or polyamide resins.

The selection of protecting groups of amino acid side claims is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butoxycarbonyl (BOC) for protecting the $\varepsilon$-amino group, in part because the BOC protecting group is readily removed by relatively mild acids such as trifluoroacetic acid (TFA), or hydrogen chloride in ethyl acetate.

The OH group of Thr and Ser may be protected by the Bzl (benzyl) group and the ε-amino group of Lys may be protected by the isopropoxycarbonyl (IPOC) group or the 2-chlorobenzyloxycarbonyl (2-Cl-CBZ) group. Treatment with HF or catalytic hydrogenation are typically employed for removal of IPOC or 2-Cl-CBZ.

For preparing cocktails of closely related peptides, see, e.g., Houghton, R.A., Proc. Natl. Acad. Sci. USA 82, 5131 (1985).

## B. Expression of PB1 in a Recombinant Expression System

It is now a relatively straightforward technology to prepare cells expressing a foreign gene. Such cells act as hosts and include E. coli, B. subtilis, yeasts, fungi, plant cells or animal cells. Expression vectors for many of these host cells have been isolated and characterized, and are used as starting materials in the construction, through conventional recombinant DNA techniques, of vectors having a foreign DNA insert of interest. Any DNA is foreign if it does not naturally derive from the host cells used to express the DNA insert. The foreign DNA insert may be expressed on extrachromosomal plasmids or after integration in whole or in part in the host cell chromosome(s), or may actually exist in the host cell as a combination of more than one molecular form. The choice of host cell and expression vector for the expression of a desired foreign DNA largely depends on availability of the host cell and how fastidious it is, whether the host cell will support the replication of the expression vector, and other factors readily appreciated by those of ordinary skill in the art.

The technology for recombinant procaryotic expression systems is now old and conventional. The typical host cell is E. coli. The technology is illustrated by treatises such as Wu, R (ed) Meth. Enzymol. 68 (1979) and Maniatis, T. et. al., Molecular Cloning: A Laboratory Manual Cold Spring Harbor 1982.

The foreign DNA insert of interest comprises any DNA sequence coding for PB1 (or fragment thereof of at least 5 amino acids in length) of the present invention, including any synthetic sequence with this coding capacity or any such cloned sequence or combination thereof. For example, PB1 peptide coded and expressed by an entirely recombinant DNA sequence is encompassed by this invention.

Vectors useful for constructing eukaryotic expression systems for the production of recombinant PB1 comprise the DNA sequence for PB1, operatively linked thereto with appropriate transcriptional activation DNA sequences, such as a promoter and/or operator. Other typical features may include appropriate ribosome binding sites, termination codons, enhancers, terminators, or replicon elements. These additional features can be inserted into the vector at the appropriate site or sites by conventional splicing techniques such as restriction endonuclease digestion and ligation.

Yeast expression systems, which are one variety of recombinant eukaryotic expression systems, generally employ Saccharomyces cerevisiae as the species of choice for expressing recombinant proteins. S. cerevisiae and similar yeasts possess well known promoters useful in the construction of yeast expression systems, including but not limited to GAP491, GAP10, ADH2, and alpha mating factor.

Yeast vectors useful for constructing recombinant yeast expression systems for expressing PB1 include, but are not limited to, shuttle vectors, cosmid plasmids, chimeric plasmids, and those having sequences derived from 2-micron circle plasmids.

Insertion of the appropriate DNA sequence coding for PB1 into these vectors will, in principle, result in a useful recombinant yeast expression system for PB1 where the modified vector is inserted into the appropriate host cell, by transformation or other means.

Recombinant mammalian expression systems are another means of producing the recombinant PND for the conjugates of this invention. In general, a host mammalian cell can be any cell that has been efficiently cloned in cell culture. Host mammalian cells useful for the purposes of constructing a recombinant mammalian expression system include, but are not limited to, Vero cells, NIH3T3, GH3, COS, murine C127 or mouse L cells. Mammalian expression vectors can be based on virus vectors, plasmid vectors which may have SV40, BPV or other viral replicons, or vectors without a replicon for animal cells. Detailed discussions on mammalian expression vectors can be found in the treatises of Glover, D.M. (ed.) "DNA Cloning: A Practical Approach," IRL 1985, Vols. I and II.

Recombinant PB1 may possess additional and desirable structural modifications not shared with the same organically synthesized peptide, such as adenylation, carboxylation, glycosylation, hydroxylation, methylation, phosphorylation or myristoylation. These added features may be chosen or preferred as the case may be, by the appropriate choice of recombinant expression system. On the other hand, recombinant PB1 may have its sequence extended by the principles and practice of organic synthesis outlined above.

## GENERAL IMMUNOLOGY

The immune response to entry of a foreign substance into the body consists of secretion by plasma cells of "antibodies" which are immunoglobulin (Ig) molecules with combining sites that recognize particular determinants on the surface of the foreign substance, or antigen, and bind specifically to them. Immunoglobulin is the generic name of various isotypes of antibodies that include IgG, IgM, IgA, IgE, and IgD. The various species of Ig have similarities and differences. For example all immunoglobulin molecules have a constant portion, i.e., highly conserved (constant) amino acid sequence, within a particular Ig subclass (e.g., IgG$_1$). This constant region is responsible for various biological effector functions (e.g., complement activation). The portion of the immunoglobulin molecule responsible for immunological specificity (i.e., the antigen combining site) is called the hypervariable or CDR region. It is made up of the variable regions of the Ig heavy and light chains. These variable regions differ in amino acid sequence according to the antigenic determinant which the Ig recognizes.

Usually, the antibody (Ab) response to an antigen (Ag) is heterogeneous. Upon injection of a body with an immunogen, the body manufactures large numbers of antibodies directed against various determinant sites on the antigen. It is difficult to separate antibodies from conventional antisera containing mixtures of antibodies. It has, therefore, long been a goal to design a continuous source of defined antibodies that recognize and combine with specific antigen determinants.

## MONOCLONAL ANTIBODY

Hybridoma technology concerns the fusion of myeloma cells with lymphocytes from animals which have been immunized with a particular antigen. The resulting hybridoma cell synthesizes monoclonal antibodies that are specific against a particular antigenic determinant. Monoclonal antibodies are beginning to replace conventional antisera in standard diagnostic kits for such procedures as the radioimmunoassay. Significant work is also being done to adapt hybridoma technology for therapeutic purposes, as for example the present application.

Some properties that flow from an ideal hybridoma cell line are (1) high cloning efficiency; (2) the ability to grow rapidly in a medium supplemented with serum; (3) no secretion of myeloma immunoglobulin (Ig); and (4) stable production of large amounts of desired, homogeneous Ig after fusion.

A typical fusion protocol for making hybridomas is as follows: (1) immunize mice or rats with a certain immunogen; (b) remove the spleens from the immunized animal and make a spleen suspension in an appropriate medium; (c) fuse the suspended spleen cells with myeloma cells; (d) dilute and culture the mixture of unfused spleen cells, unfused myeloma cells and fused cells in a selective medium which will not support growth of the unfused myeloma cells or spleen; (e) evaluate the supernatant in each well or dish containing hybridoma for the presence of antibody to the immunogen; and (f) select and clone hybridomas producing the desired antibodies. Once the desired hybridomas has been selected and cloned, the resultant antibody is produced by in vitro culturing of the desired hybridoma in a suitable medium. Mouse hybridomas are well known. See, for example, Milstein et al., Nature 266, 550 (1977); Galfie et al., Meth. Enzymol. 73, 3 (1981); U.S. Patent 4,757,018.

Applicants found that mice will not respond to PB1, the antigen of the present invention. The rat system was then used as the alternative method of producing monoclonal antibodies. Despite the known difficulties inherent in the rat hybridoma technology, applicants have succeeded in isolating and characterizing 4 rat monoclonal antibodies specific for the antigen desired, PB1 each having capacity to neutralize HIV.

The critical feature of the rat fusion protocol in the present invention is the ratio of rat spleen cells to rat myeloma cells. Applicants have discovered that a ratio of 1.5 to 2.5 substantially facilitates production of rat monoclonals. The preferable ratio is about 2.0 rat spleen cells for each rat myeloma cell. In contrast, a ratio of about 5 is required by De Clerco et al. Meth. Enzymol. 121, 234 (1986).

The use of MRC-5 cells as a feeder layer for the fusions is preferred but not critical. For a description of these cells, see Long et al. J. Imm. Methods 86, 89 (1986). A variety of other feeder cell layers are feasible substitutes.

The preferred rat myeloma is Y3-Ag 1.2.3. Other rat myelomas useful for making rat monoclonal antibodies include but are not limited to LOUIR983F (a non-secretor); 210-RCY3-Ag1; or YB2/3.0 Ag 20.

Two separate protocols for producing rat monoclonal antibodies to PB1 are exemplified in this application. It will be understood that further variations or modifications of these specific experiments are well within routine skill of those familiar with the art, with the exceptions noted above.

Purification of the rat monoclonals of the present invention follows in general the principles and practice of the well-known art of isolating antibodies. A recent discussion on purification of rat monoclonal antibodies is provided by Bazin et al., Meth. Enzymol. 121, 638 (1986), which publication is hereby incorporated by reference.

## COMPOSITION FOR ADMINISTRATION

The rat monoclonal antibodies of the present invention can be incorporated into conventional pharmaceutical compositions or formulations for use in treating patients with AIDS or ARC, or for the prophylaxis of individuals at risk of these diseases. The composition or formulation should contain a therapeutic or prophylactic amount of one or more of the rat monoclonal antibodies of this invention, together with a pharmaceutically-acceptable carrier. Suitable carriers are any compatible, non-toxic substance appropriate for delivering the monoclonal to the patient, including but not limited to isotonic saline, sterile water, alcohol, fats, waxes, 10% maltose, human serum albumin, glycine or other similar diluents, salts, carriers or vehicles.

The monoclonal antibodies of the present invention may be administered as a cocktail of antibodies for the purpose of treating or protecting against more than one HIV serotype. Alternatively or additionally, the monoclonal antibodies may be effectively administered, whether at periods of pre-exposure or post-exposure, as combinations with effective amounts of HIV-specific antivirals, immunomodulators, antibiotics or vaccines.

The monoclonal antibodies and pharmaceutical formulations thereof in this invention are effectively administered by oral or parental routes. Parental administration is preferred, particularly an intravenous mode. The concentration of monoclonal antibody in these formulations can range from about 0.5% to more than 25% by weight.

For intramuscular injection, a typical dose of between about 10 mg and about 100 mg monoclonal antibody in 1 ml isotonic saline would be effective. Therapeutically effective doses range from about 0.5 mg to about 100 mg monoclonal antibody per kilogram of body weight. Prophylactically effective dosages range from about 0.05 mg to about 20 mg per kilogram of body weight.

It will be understood that the specific dose level and frequency of dosage for any particular patient may be varied and will depend upon a variety of factors including the activity of the specific monoclonal antibody employed, the metabolic stability and length of action of that antibody, the age of the patient, body weight, general health, sex, diet, mode and time of administration, rate of excretion, drug combination, the severity of the particular condition, and the host undergoing therapy.

## ATCC DEPOSITS

On or before the U.S. filing date of the present application, samples of each rat hybridoma 20G3-2, 24D3-2, 28G12-2, and 1G7-2 were deposited at the American Type Culture Collection (ATCC), 12301 Parklawn Drive, Rockville MD 20852. Culture access designations are HB10541, HB10542, HB10543, and HB10540, respectively. These deposits will be maintained in the ATCC for at least 30 years and will be made available to the public upon the grant of a patent disclosing them. It should be understood that the availability of a deposit does not constitute a license to practice the subject invention in derogation of patent rights granted by government action.

## EXAMPLE 1

### Immunization Protocol for Fusion Partner

Rats were immunized with an immunogen which contains major type-specific neutralizing determinant of the HIV (IIIb isolate) envelope protein. The immunogen, termed PB1, was derived from recombinant E. coli and was composed of the carboxy-terminal half of the envelope protein. Following the immunization series, the rats were found to have developed anti-PB1 antibody activity. The protocol follows.

Osborne/Mendel rats were immunized each with 50 mcg of PB1 in a primary immunization, boosted on day 14 (100 mcg/rat) and on day 28 (500 mcg/rat), and bled on day 25 and day 39.

Immunization protocols are dependent on antigenicity of the material utilized for immunization. Both single and multiple dose immunization protocols have given rise to successful hybridoma production. Assay of rat serum for polyclonal antibody response assures sensitized spleen cells for fusion. An intravenous priming dose of antigen was injected 3-4 days prior to the actual fusion date to assure an abundance of blasting cells.

## EXAMPLE 2

### Maintenance of Fusion Partner Myeloma

1. Strain of cells. Rat myeloma Y3-Ag 1.2.3 ("Y-3") was selected for its hypoxanthine guanine phosphoribosyl transferase deficiency.

2. <u>Culture of cells</u>. Cells were planted at 2 x $10^5$ cells per ml in Ham's F-12/Dulbecco's MEM (with L-Glutamine) and high glucose + 10% fetal bovine serum and then either gassed with 5% $CO_2$ and air or incubated in a 5-7% $CO_2$ chamber at 36°C. Cells reach confluency in approximately 4 days and require subcultivation into fresh medium. The cells are sensitive to pH, cell density and glutamine concentration. Cells should not be allowed to grow at densities greater than 2.0 x $10^6$ cells par ml.

3. <u>Maintenance of genetic selection</u>. Every 6-8th passage the Y-3 celles were cultured with 8-azaguanine, which selects for the HGPRT-deficient cells. Cells should be checked prior to fusion for the deficiency by culturing in medium containing aminopterin and α-thymidine. Check in one week to insure no growth.

4. <u>Freezing of cells</u>. Y-3 cells are frozen at a concentration of 1.5-2 x $10^6$ cells/ml in 85% Eagle's BME (Earle's Salts), 10% fetal bovine serum, and 5% DMSO at a volume of 3 ml/ampoule; freeze in liquid nitrogen.

5. <u>Preparation for fusion</u>. Y-3 cells should be used no closer than one subculture from 8-azaguanine treatment and no more than 7 subcultures from it. On the day prior to fusion, the cells should be resuspended in fresh medium to insure active cell division. It is advisable to perform a viable cell count (trypan blue) at this time as an indication of cell concentration and viability less than 95 %. Cells should be in logarithmic growth phase for a minimum of 10 days prior to use.

## EXAMPLE 3

## FUSION WITH RAT MYELOMA LINE

In general, pipettors should be slow speed. Addition of cell suspensions, e.g. newly fused cells, to feeder cultures should not be performed dropwise but instead by contacting the filled pipettor tip to the meniscus of the feeder cell dish.

### 1. Fusion Protocol A

Spleens of rats immunized with PB1 were disrupted with a 70 micron-hole screen and counted after collecting the cells and washing the screen with serum-free HT/R [213 ml HAMS F12 of Gibco + 213 ml DMEM of Flow + 50 ml NCTC 109 (a vitamin supplement) + 5.0 ml 100 x HT stock (100 x HT stock is 408 mg hypoxanthine + 114 mg thymidine per 300 ml $H_2O$) + 5.0 ml 100 x OPI stock (1500 mg oxalacetic acid, 500 mg pyruvic acid, 12.7 mg bovine insulin (containing 25.5 units/mg) per 100 ml $H_2O$) + 10 ml 200 mM L-glutamine + 5 ml P/S (10,000 units of Penicillin G/ml and 10,000 micrograms/ml Streptomycin sulfate)]. The total spleen cell count from the screen was 1.0 x $10^9$. The spleen cells were washed three times with serum-free HT/R.

Fusion partner Y-3-Ag 1.2.3 rat myeloma cells were counted (8.1 x $10^5$/cc), washed three times in serum-free HT/R, and combined with the spleen cells in a 1:2 ratio. The combined cell mixture was centrifuged 1000 RPM, for 10 minutes to pellet the cells, the supernatant aspirated. Aliquots of 4.6 ml of PEG (50% w/v in Hepes, average MW = 1500) were added to each cell pool (each about 200 microliters), slowly over 1 minute, stirred 1 more minute, then centrifuged at 165 x g for 5-6 minutes. The PEG was aspirated and the cell mixture was resuspended to 5.0 x $10^6$ spleen cells /ml in HT/R 20% FCS [165 ml HAMS F12 + 165 ml DMEM + 50 ml NCTC 109 + 100 ml FCS + 5.0 ml HT stock + 5ml 100 X OPI stock + 10 ml 200 mM L-glutamine + 1 ml P/S]. The addition of FCS interferes with further PEG-induced cell fusion. Aliquots of 0.1 ml of cell suspension were planted in each well of a 96 well microliter plate, each well being previously coated with a feeder cell layer of irradiated MRC-5 cells. Plates were incubated at 37°C, 95% humidity, 5-8% $CO_2$. Wells refed the following day with HAT medium, [HT/R 20% FCS + 80 nM aminopterin], all other subsequent feedings were with HT/R 20% FCS. Thereafter fast growers were selected out for regrowth, or subcloned. Replating was also performed by limiting dilution to assure monoclonal outgrowth, as individual cases necessitated.

### 2. Fusion Protocol B

A feeder layer of human fibroblast MRC-5 cells was suspended in HT/R20% FCS at 1-4 X $10^6$ cells per ml in a plastic conical tube and then irradiated with 10,000 rads cobalt 60 (the time interval between trypsinization, irradiation, and planting should not exceed two hours). The irradiated cells were diluted in HT/R20% FCS to 80,000 cells/ml and 0.1 ml/well was planted a minimum of 24 hours prior to use, and preferably 72 hours prior to use.

Rat spleens(s) were collected after orbital bleeding and sacrificing by $CO_2$ asphyxiation. Under aseptic conditions, the spleen(s) was removed and placed in 30 ml serum-free HT/R medium at 25°C. Sterility testing of transport medium was also performed.

In a laminar flow hood, most of the medium was aspirated off the spleens and transferred to a 60 mm petri dish + 5 cc serum-free HT/R medium. With forceps and plastic scraper*, cells from the spleens were teased apart. The resulting suspension was placed in a 15 ml conical tube, the petri dish washed with 5 ml serum-free HT/R medium to collect all the cells and allow the pooled suspension to settle approximately 1 min. to remove the large particles. The supernatant fluid was transferred to a 15 ml round bottom tube and centrifuged at 275 xg for 10 minutes.

*(Alternately spleens may be pushed through a Bellco cellector with 80 micron mesh screen by a sterile plastic syringe barrel, then washed with HT sera-free medium.)

Supernatant fluid was discarded and the cells gently resuspended in 30 ml serum-free HT/R medium.

A viable cell enumeration was performed with trypan blue. Various antigens appeared to affect spleen cell viability. One spleen may yield from $5.0 \times 10^7$ to $3.4 \times 10^8$ cells.

Spleen cells were mixed 2:1 with viable Y-3 cells in 50 ml conical tubes. The mixture was washed twice with 50 ml serum-free HT/R at 275 xg for 10 minutes. A third wash was performed in a 15 ml round bottom tube with 10ml serum-free HT/R and the medium aspirated from a myeloma-spleen cell pellet.

PEG 1000 was diluted to 35% (w/v) with serum-free HT/R medium, DMSO then added to give 5% (V/V) final concentration, and the resulting "PEG" maintained at 37°C. The myeloma-spleen cell pellet was resuspended by gently tapping the tube and adding the PEG ($0.8$ cc/$1.8 \times 10^8$ spleen cells) dropwise with gentle stirring over one minute. Gentle stirring was continued for an additional minute, then the cells centrifuged at 165 xg. The PEG was carefully aspirated and the cells resuspended with gentle tapping. Total PEG contact time (stirring and centrifugation) should be 6-8 minutes.

The cells were resuspended to a concentration of $3.3 \times 10^6$ spleen cells per ml in HT/R 20% FCS and plated 0.1 cc per well in sterile microtiter plates containing irradiated MRC-5 fibroblasts (8000/well). A flask of myeloma cells was planted as a control for aminopterin efficacy.

Cells were incubated at 37°C 5-8% moist $CO_2$ for 24 hours.

HAT medium was prepared with 2X aminopterin (2 ml aminopterin stock/100 ml serum-free HT/R or HT/R 20% FCS) to yield HAT medium or HAT/20% FCS medium, respectively.

Cells were cultured at 37°C in 5% $CO_2$ for 24 hours, then 0.1 cc HAT/20% FCS/well was added. Further culture of cells was performed by changing medium every 3-4 days. Aliquots of 0.1 cc medium were removed and replaced with 0.1 cc HAT/20% FCS until the myeloma control flask indicated no viable myelomas (usually 10-14 days). Culturing was continued with HT/R 20% FCS. Antibody production should be assayed when cells are at least 20-25% confluent.

Hybridomas producing desired antibodies were transferred from the microtiter plates when they reached 60-70% confluency. Hybridomas were transferred to 24 well tissue culture plates and subsequently to larger growth flasks. HT/R 20% FCS is the preferable culture medium. Optimal planting levels are $1.5\text{-}2.0 \times 10^5$ cells per ml and should not exceed $2.0 \times 10^6$ cells per ml.

EXAMPLE 4

Selection of Lines Producing Monoclonal Antigen-Specific Antibody

Supernatants of all wells were assayed for antigen-specific antibody by a modified ELISA technique, as follows. Vinyl 96 well plates were incubated overnight at 5°C (or for 2 hours at 37°C) with peptide antigen (PB1 diluted in 0.5M Carbonate buffer pH 9.5), covered with parafilm to prevent drying. After such absorption, the plates were emptied and washed three times with PBS, then to each well was added 200 $\mu$l of non-specific binder containing 3% Nonfat Dry Milk and 5% FCS in PBS. Incubation with the non-specific binder was performed at room temperature for 60 minutes, after which the wells were emptied and washed once with PBS.

Aliquots of cell well supernatants containing monoclonal antibody to be tested were diluted in PBS and added in 100 $\mu$l volumes to each well. The plates were incubated at room temperature for 2 hours to promote specific binding of monoclonal antibody to PB1 absorbed on the solid-phase substrate. The plates were emptied, washed once with non-specific binder (supra), then three times with 0.05% TWEEN in PBS.

Bound antibody was measured with a goat anti rat IgG conjugated to alkaline phosphatase ("AP-goat anti-hIgG") as follows:

Aliquots of 100 $\mu$l were added to each well of a 1:250 dilution of AP-goat anti-hIgG (1000 micrograms/ml) in PBS, and thereafter incubated at room temperature for 3 hours. Plates were washed three times with 0.05% TWEEN in PBS, then three times with distilled water. A color-producing substrate p-nitrophenyl phosphate was added in 100 $\mu$l aliquotes of 5 mg/ml substrate buffer (prepared by combining 10 ml diethanolamine and 10 mg $MgCl_2\text{-}6H_2O$, diluting to 100 ml with distilled $H_2O$, adjusted to pH 9.8 with 1.0 N HCl). The color reaction is stopped with 100 $\mu$l 3N NaOH.

In a typical 96-well plate, several wells tested positive by modified ELISA.

EP 0 479 454 A1

EXAMPLE 5

Selection of Hybridoma Lines Producing HIV-neutralizing Monoclonal Antigen-specific Antibody

Four hybridoma lines testing positive by modified ELISA were subjected to HIV neutralization assays. In a 96 well cell culture plate, test supernatants were diluted serial twofold in media (RPMI 1640 + 10% FBS). Virus (HIV H9IIIb) was added to each well, then mixed with antibody by repeated pipetting. After incubation for one hour at room temperature, about 50,000 MT-4 cells are added to each well, incubated at 37°C for 7 days. Aliquots of MTT metabolic stain [3-(4,5-dimethylthiazol-2-yl)-2,5-diphenyltetrazolium bromide) are added, incubated at 37°C for 2-4 hours. Half the volume of each well is removed, then replaced with acid isopropanol/0.5% (W/V) SDS. The color produced is blue or purple.

Four hybridoma lines were found to neutralize HIV: 20G3-2, 24D3-2, 28G12-2 and 1G7-2.

Each was found to be an IgG monoclonal antibody. Two of the four hybridoma lines are particularly potent in in vitro neutralizing activity against IIIb isolate of HIV.

EXAMPLE 6

Cloning of Hybridomas

A feeder layer of fibroblast cells (MRC-5) was suspended in hybridoma culture medium (HT/R 20% FCS) at 1 - 4 x 10$^6$ cells/ml in a plastic centrifuge tube. The fibroblasts were irradiated with 10,000 rads cobalt 60. (The time interval between trypsinization, irradiation and planting should not exceed 2 hrs.) Cells were then diluted to 80,000/ml in HT/R 20% FCS and planted 0.1 ml per microtiter well. Twenty-four hours was the minimum time required for the fibroblasts to condition the medium.

The hybridomas were harvested in log phase and resuspended to a cell suspension of 2 cells/ml in HT/R 20% FCS. Aliquots of 0.1 ml cell suspension were replated in each new well. Cells were refed whenever the medium became acidic or at a mininum once a week. Each assumed clone was re-cloned at least once to confirm a single cell clone.

Production of antibody by monoclonal cells in significant quantity is accomplished by large-scale tissue culture or collection of ascites fluid.

While the foregoing specification teaches the principles of the present invention, with examples provided for the purpose of illustration, it will be understood that the practice of the invention encompasses all of the usual variations, adaptations, modifications, deletions or additions, as come within the scope of the following claims and its equivalents.

**Claims**

1. Rat monoclonal antibody specific for PB1, said antibody having HIV neutralizing activity.

2. A hybridoma cell line producing rat monoclonal antibody specific for PB1, said antibody having HIV neutralizing activity.

3. A hybridoma cell line of claim 2, designated as 20G3-2, 24D3-2, 28G12-2, or 1G7-2.

4. A rat monoclonal antibody produced by hybridoma cell lines 20G3-2, 24D3-2, 28G12-2, or 1G7-2, said antibody having HIV neutralizing activity.

5. A pharmaceutical composition comprising a rat monoclonal antibody produced by hybridoma cell lines 20G3-2, 24D3-2, 28G12-2, or 1G7-2 and a pharmaceutically acceptable carrier thereof.

6. The use of a rat monoclonal antibody produced by hybridoma cell lines 20G3-2, 24D3-2, 28G12-2, or 1G7-2 for the preparation of a medicament for the treatment of infection by HIV.

7. The use of a rat monoclonal antibody produced by hybridoma cell lines 20G3-2, 24D3-2, 28G12-2, or 1G7-2 for the preparation of a medicament for passively immunizing against HIV.

11

**European Patent Office**

# EUROPEAN SEARCH REPORT

Application Number

EP    91 30 8585

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,,, P | Biological Abstracts Vol.91 Reference 40156 (1991) cites AKERBLOM,Lennart et al.:?Neutralizing cross-reactive and non-neutralizing monoclonal antibodies to HIV-1 gp 120' AIDS (PHILA) 4(10) 953-960 (1990) --- | 1-7 | C07K15/00 C12P21/08 |
| Y | AIDSLINE Reference 8975182 cites PUTNEY S. et al 'HIV subunit vaccine development using recombinant proteins and synthetic peptides'Fourth International Conference On Aids.Book 1.June12-16, 1988,Stockholm,Sweden;p.161 (1988) --- | 1 | |
| Y | AIDSLINE Reference 88748279 cites PUTNEY SD et al. 'Expression of HTLV-III envelope proteins in Escherichia Coli and their induction of neutralizing antibodies.' Vaccines 87.Modern approaches to new vaccines:prevention of AIDS and other viral,bacterial,and parasitic diseases.Chanock RM et al.,eds.New York, Cold Spring Harbor Laboratory, P. 256-9, (1987) --- | 1 | |
| Y | Biological Abstracts Vol.86, Reference 33836 (1988) cites MATSUSHITA S. et al. 'Characterisation of a human immunodeficiency virus neutralizing monoclonal antibody and mapping of the neutralizing epitope'.J. Virol. 62(6)2107-2114 (1988) ----- | 1 | |

**TECHNICAL FIELDS SEARCHED (Int. Cl.5)**

C07K
C12P

The present search report has been drawn up for all claims

| Place of search | Date of completion of the search | Examiner |
|---|---|---|
| THE HAGUE | 20 DECEMBER 1991 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)